# EUROPEAN PATENT SPECIFICATION

(11) **EP 3 632 854 B1**
(45) Date of publication and mention of the grant of the patent: **22.12.2021**
(21) Application number: 19200120.4
(22) Date of filing: 27.09.2019
(51) Int. Cl.: C02F 1/00, G01N 27/06, G01N 33/18

(54) **A METHOD FOR MEASURING WATER QUALITY AND A METHOD FOR OPERATING A SYSTEM ALLOWING FOR PURIFICATION AND RECYCLING OF WATER OR SEPARATION OF WATER**
VERFAHREN ZUR MESSUNG DER WASSERQUALITÄT UND VERFAHREN ZUM BETRIEB EINES SYSTEM ZUR ERMÖGLICHUNG DER REINIGUNG UND WIEDERVERWENDUNG VON WASSER ODER DER TRENNUNG VON WASSER
PROCÉDÉ DE MESURE DE LA QUALITÉ DE L'EAU ET PROCÉDÉ DE FONCTIONNEMENT D'UN SYSTÈME PERMETTANT DE PURIFIER, DE RECYCLER OU DE SÉPARER DE L'EAU

(30) Priority: 02.10.2018 SE 1851184
(43) Date of publication of application: 08.04.2020
(73) Proprietor: Orbital Systems, 211 20 Malmö (SE)
(72) Inventor: FRIBERG, Markus, 211 33 MALMÖ (SE)
(74) Representative: AWA Sweden AB

(56) References cited:
- WO-A1-2015/094107
- US-A- 5 581 189
- US-A1- 2001 029 435

## Description

### Field of the invention

The present invention relates to a method for measuring water quality in a system, and to a method for operating a system allowing for purification and recycling of water or separation of water.

### Technical Background

Systems and methods for operating systems intended for allowing for purification and recycling of water or separation of water are known. Moreover, such systems involving multiple conductivity sensors are also described. For instance, in WO2015/094107 there is disclosed a hybrid device allowing purification and either recycling of water or discarding of water, wherein said hybrid device comprises a recirculation loop, a filter system and multiple sensors, wherein the multiple sensors are conductivity sensors, wherein the hybrid device also comprises a micro-processor and wherein the multiple sensors are connected to the micro-processor.

In its most general aspect, the present invention is directed to a method for measuring water quality in a system comprising multiple conductivity sensors. One aim of the present invention is to provide a method which provides higher accuracy in the measurement of a system involving multiple conductivity sensors, including using this measurement as an indicator for water quality.

### Summary of the invention

The stated purpose above is achieved by a method, as defined in claim 1, for measuring water quality in a system comprising a sensor system with multiple conductivity sensors located close to each other, including a conductivity sensor directed to measuring water quality, said method comprising a discharge and sampling sequence for each conductivity sensor, wherein only one conductivity sensor is measured or sampled at a time so that when one is undergoing a discharge and sampling sequence, then the other one(s) is(are) set in rest position, corresponding to a high impedance state of sensor electrodes to avoid interference with the active conductivity sensor.

Moreover, the method according to the present invention is also directed to a method, as defined in claim 2, for operating a system allowing for purification and recycling of water or separation of water, said system comprising a sensor system comprising multiple conductivity sensors located close to each other and also a control system, where at least one conductivity sensor is directed to measuring water quality, wherein the sensor system gives input to the control system with respect to a selection decision of either recycling of water in the system or separation of water from the system in at least one separation point, said method also comprising a discharge sequence for each of said conductivity sensors.

As mentioned above, there are systems today using electrodes in connection to water measurement. For instance, US 5,581,189 discloses a water purity testing system using electrodes and having reversing polarity. Moreover, US 2001/029435 A1 discloses a method of measuring representations of electrical conductivity of an aqueous solution which compensates for polarization, said method involving generating a DC pulse to induce a current between first and second electrodes positioned within the solution, sampling a voltage at the first electrode at a sequence of at least three predetermined time intervals and generating a corresponding sequence of at least three voltage values, calculating a value corresponding to the voltage at the first electrode at a time contemporaneous with the generation of the DC pulse, and generating an output signal as a function of the calculated value.

The method according to the present invention has several advantages. Without the use of the discharging sequence of the method according to the present invention, a net ionization occurs. This net ionization gives rise to a build-up or electroplating on the sensor electrodes. The long term effects of this is fatigue of the sensor electrodes, which may lead to that these units stop working. Furthermore, also the actual measurement correctness will be affected over time. The method according to the present invention solves this issue by involving a discharge sequence. Based on the above, the method according to the present invention provides the possibility of intermittent measurement.

The alternative to this is to use continuous alternating excitation (bipolar excitation), however this would not work when using multiple conductivity sensors. The method according to the present invention, however, is for a sensor system comprising multiple conductivity sensors, as is further disclosed below. By use of the method according to the present invention it is possible to prevent the electroplating and build-up on the sensor electrodes. As such, the electrodes, often incorporated as an electrode pair, of each conductivity sensor are made more durable and the expected life time in a system like this is prolonged. Moreover, the accuracy in the measurement is maintained at a high level also over time. Furthermore, the method according to the present invention also enables to have electric conductivity sensors located close to one another without having to be isolated. This is also a clear benefit according to the present invention.

The present invention refers to a method for measuring water quality in a system comprising a sensor system with multiple conductivity sensors, said method comprising a discharge sequence for each of the multiple conductivity sensors. This implies that the method according to the present invention, and also any of the embodiments disclosed below may be utilized in a method involving measuring water quality in a system comprising a sensor system with multiple conductivity sensors located close to each other, i.e. also in such systems not being water recirculation systems.

### Specific embodiments of the invention

Below specific embodiments of the present inventions are discussed.

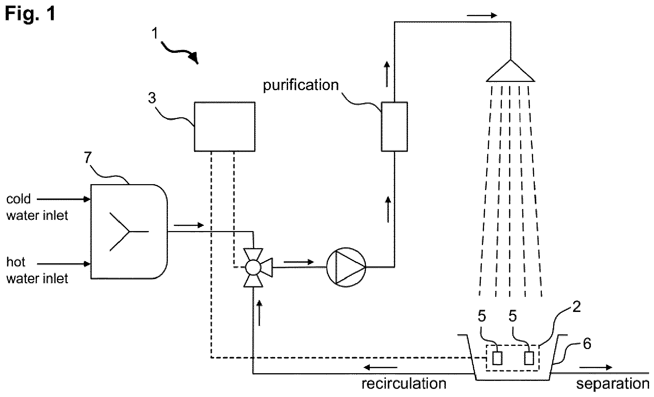

According to one specific embodiment of the present invention, the sensor system comprises multiple conductivity sensors which are positioned in close proximity to said at least one separation point. As the conductivity measure may be an indication of water quality it may be of interest to have this measurement close to the separation point where water is either separated off or recirculated. It should be noted that the positioning of the actual conductivity sensors may be in different positions and also several positions in the system. According to one embodiment, the multiple conductivity sensors are positioned in a drain of the system allowing for purification and recycling of water or separation of water. As seen in fig. 1, the drain may be in a position close to or at said at least one separation point. As said, also other positions may very well come into play. According to one specific embodiment of the present invention, said multiple conductivity sensors are positioned in a water tank of the system allowing for purification and recycling of water or separation of water.

The positioning may also be chosen based on the intent. According to one specific embodiment of the present invention, each conductivity sensor is directed to measuring level of water or water quality. Both water quality and level may be relevant to measure when the sensors are provided in a drain. The same is actually also valid for a water tank having such sensors inside, however in this case level might also be the only thing measured. Moreover, it should also be noted that the multiple conductivity sensors may be arranged at different positions in the system.

Also the actual discharge sequence of the method according to the present invention may vary. According to one specific embodiment of the present invention, the discharge sequence for each specific sensor of said multiple conductivity sensors comprises exciting the specific sensor with one polarity side, sampling the specific sensor, exciting the specific sensor with the other polarity side and then discharging the specific sensor. It should be noted that only one conductivity sensor is measured or sampled at a time, and therefore there is a small pause between each individual discharge sequence. To measure one at a time is important as conductivity sensors located close to each other may otherwise affect each other, and thus affect the measurement. This also implies that when having two electric conductivity sensors in a unit, then when one is undergoing a discharge and sampling sequence, then the other is set in rest position. In rest position the two sensor electrodes are set to be in a high impedance state to avoid interference with the active electrical conductive (EC) sensor. Moreover, and as understood, the electric conductivity sensor in the discharge and sampling sequence is finally excited with the remaining polarity side before a new sensor may go into the sequence.

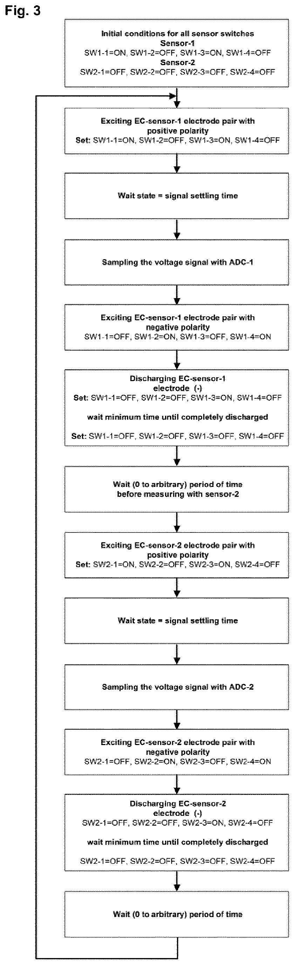

As seen in fig. 3, according to one specific embodiment, the first step of exciting is performed with positive polarity and the second step of exciting is performed with negative polarity, however the other way around is of course totally possible. Furthermore, according to yet another specific embodiment, the exciting steps are performed in a time range of 20 - 500 µs, such as in a time range of 50 - 200 µs.

As hinted above, the system according to the present invention comprises multiple electric conductivity sensors. According to one specific embodiment of the present invention, the system comprises at least three sensors of which one is a low water level sensor, one is a high water level sensor and a third is a conductivity measure sensor, and wherein the method comprises first a discharging sequence on the low water level sensor, then a discharging sequence on the conductivity measure sensor and finally a discharging sequence on the high water level sensor. All of these sensors may be provided in a drain of the system, however the same setup of sensors may be arranged in a water tank or in another localization of the system.

### Detailed description of the drawings

In fig. 1 there is shown a system 1 allowing for purification and recycling of water or separation of water, where the system comprises a sensor system 2 which in this case is positioned in a drain 6 and which comprises two conductivity sensors 5. The sensor system 2 may in this case be seen as a sensor unit. It should be noted that the sensor system 2 of course may comprise other sensors, such as more conductivity sensors 5 or other sensor types. For instance, in the box where purification is performed other sensors may be involved. As an example, UV or turbidity sensors may be involved in the system. It should also be noted that also other sensors, such as temperature, pressure or flow etc., may of course also be part of the sensor system 2.

Furthermore, as seen in fig. 1, the water recirculation system 1 is a shower in this case, but can be of a different type. Non-limiting examples are sinks, toilets, washing machines, dish-washers or combinations thereof. The used water in the shower is collected in the drain 6 where the conductivity sensors 5 indicate the conductivity measure and sends the information to the control system 3. It is in this step where the method according to the present invention comes into play. It should, however, be noted that the conductivity sensors 5, in this case provided as a sensor unit with two individual conductivity sensors 5, may be arranged in different positions in the system 1, e.g. in the water tank 7 or elsewhere. It is, however, preferred that at least two multiple conductivity sensors 5 are located in close proximity to a separation point. It is in this separation point where a decision is made by the control unit 3 with reference to if used water should be separated off or sent to recirculation / recycling. It should be noted that the system 1 may comprise several separation points so that water of different quality levels may be sent to different receiving sources. To separate into different water quality fractions may also be possible to perform only from one separation point.

Moreover, it should be noted that a system 1 may comprise many other sensors also, such as other conductivity sensors, e.g. provided in the water tank 7 or elsewhere in the system, and other water quality sensors, such as turbidity sensors or UV sensors, where the latter may function both as a water quality sensor but can also functions as a quality sensor for a UV source. Moreover, also other sensors, such as e.g. temperature, pressure and flow etc. may of course also be arranged in the system 1.

The water sent to recirculation, i.e. water having a quality level which allows for recirculation, is pumped up through a purification unit. Before this, it may be mixed with inlet water coming from a water tank 7. This water tank 7 is connected to a mixing point and a valve for inlets of cold and hot water. The water tank 7 may be equipped with an air gap for hygenization reasons and also be connected to an outlet valve. From the water tank 7 fresh inlet water may be mixed with recirculation water when the outlet valve is open. Moreover, the purification may comprise different purification sources, such as e.g. an UV unit, combined UV and heating and/or a filter unit, or combinations thereof. Also other techniques are possible. After the purification, then the water flow is further flowed to be reused, such as in this case to be flowed out from the nozzle of the shower.

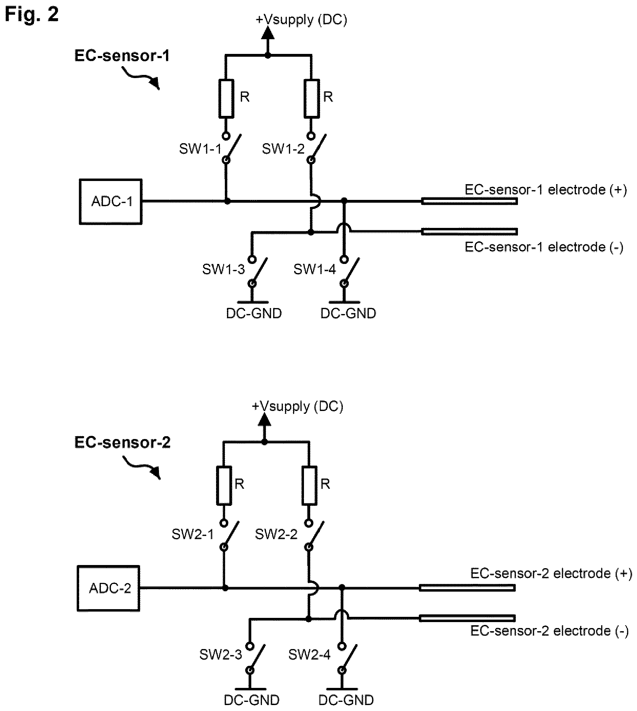

In fig. 2 there is shown a possible setup for one EC (electrical conductivity) sensor, called EC sensor 1, according to the present invention as well as a corresponding set up for a second EC sensor, called EC sensor 2. In addition to what is already described in fig. 2, the following abbreviations also apply:
SW1-1 = Electrical switch no 1 in EC sensor 1 H bridge
SW2-2 = Electrical switch no 2 in EC sensor 2 H bridge
etc.
R = Arbitrary resistor
ADC-1 = Analogue to Digital Converter that samples EC-sensor 1 output signal

In fig. 3 there is shown one possible discharge sequence for two conductivity sensors according to one embodiment of the present invention. Each of said at least one conductivity sensor 5 is subjected to the sequence steps of first exciting the specific sensor 5 with one polarity side, in this case on the positive side, then sampling, after that exciting on the other polarity side, i.e. on the negative side in this case, and finally discharging. When this is first performed on a first conductivity sensor 5, then all of the steps are performed on a second conductivity sensor 5. It should be noted that the system 1 may comprise several multiple conductivity sensors 5 where then a third and a fourth sequence and so on are performed subsequently. Moreover, a system 1 according to the present invention may comprise several units of multiple conductivity sensors 5 where a first unit first undergoes a first and second sequence, and then later such sequences are performed in another unit comprising at least two conductivity sensors 5.

## Claims

1. A method for measuring water quality in a system (1) comprising a sensor system (2) with multiple conductivity sensors (5) located close to each other, including a conductivity sensor (5) directed to measuring water quality, said method being **characterized by** comprising a discharge and sampling sequence for each conductivity sensor (5), wherein only one conductivity sensor (5) is measured or sampled at a time so that when one is undergoing a discharge and sampling sequence, then the other one(s) is(are) set in rest position, corresponding to a high impedance state of sensor electrodes to avoid interference with the active conductivity sensor (5).

2. A method for operating a system (1) allowing for purification and recycling of water or separation of water, said system (1) comprising a sensor system (2) and a control system (3), wherein the sensor system (2) comprises multiple conductivity sensors (5) located close to each other, including a conductivity sensor (5) directed to measuring water quality, wherein the water quality in the system (1) is measured with said sensor system (2) using a method according to claim 1, and wherein the sensor system (2) gives input to the control system (3) with respect to a selection decision of either recycling of water in the system (1) or separation of water from the system (1) in at least one separation point.

3. The method according to claim 2, wherein the multiple conductivity sensors (5) are positioned in close proximity to said at least one separation point.

4. The method according to any of claims 2-3, wherein said multiple conductivity sensors (5) are positioned in a drain (6) of the system (1) allowing for purification and recycling of water or separation of water.

5. The method according to any of claims 2-3, wherein at least one conductivity sensor (5) is further positioned in a water tank (7) of the system (1) allowing for purification and recycling of water or separation of water.

6. The method according to any of claims 1-5, wherein each of said multiple conductivity sensors (5) are directed to measuring level of water or water quality.

7. The method according to any of claims 1-6, wherein the discharge sequence for each specific sensor (5) of said multiple conductivity sensors (5) comprises exciting the specific sensor (5) with one polarity side, sampling the specific sensor (5), exciting the specific sensor (5) with the other polarity side and then discharging the specific sensor (5).

8. The method according to claim 7, wherein the first step of exciting is performed with positive polarity and the second step of exciting is performed with negative polarity.

9. The method according to claim 7 or 8, wherein the exciting steps are performed in a time range of 20 - 500 µs.

10. The method according to any of claims 1-9, wherein the system (1) comprises at least three sensors (5) of which one is a low water level sensor, one is a high water level sensor and a third is a conductivity measure sensor, and wherein the method comprises first a discharging sequence on the low water level sensor, then a discharging sequence on the conductivity measure sensor and finally a discharging sequence on the high water level sensor.

## Patentansprüche

1. Verfahren zum Messen der Wasserqualität in einem System (1), das ein Sensorsystem (2) mit mehreren Leitfähigkeitssensoren (5) umfasst, die nah beieinander angeordnet sind und die einen Leitfähigkeitssensor (5) umfassen, der auf ein Messen der Wasserqualität abzielt, wobei das Verfahren **dadurch gekennzeichnet ist, dass** es eine Entlade- und Abtastsequenz für jeden Leitfähigkeitssensor (5) umfasst, wobei zu einem Zeitpunkt nur ein Leitfähigkeitssensor (5) gemessen oder abgetastet wird, so dass, wenn einer eine Entlade- und Abtastsequenz durchläuft, dann der (die) andere(n) in eine Ruheposition versetzt ist (sind), die einem Zustand hoher Impedanz der Sensorelektroden entspricht, um eine Störung des aktiven Leitfähigkeitssensors (5) zu vermeiden.

2. Verfahren zum Betreiben eines Systems (1), das die Reinigung und Wiederverwendung von Wasser oder die Trennung von Wasser ermöglicht, das System (1) ein Sensorsystem (2) und ein Steuerungssystem (3) umfassend, wobei das Sensorsystem (2) mehrere Leitfähigkeitssensoren (5) umfasst, die nah beieinander angeordnet sind und die einen Leitfähigkeitssensor (5) umfassen, der auf ein Messen der Wasserqualität abzielt, wobei die Wasserqualität in dem System (1) unter Verwendung eines Verfahrens nach Anspruch 1 mit dem Sensorsystem (2) gemessen wird und wobei das Sensorsystem (2) eine Eingabe an das Steuerungssystem (3) hinsichtlich einer Auswahlentscheidung macht, entweder Wasser in dem System (1) wiederzuverwenden oder Wasser aus dem System (1) an mindestens einem Trennpunkt zu trennen.

3. Verfahren nach Anspruch 2, wobei die mehreren Leitfähigkeitssensoren (5) in enger räumlicher Nähe zu dem mindestens einen Trennpunkt positioniert sind.

4. Verfahren nach einem der Ansprüche 2 bis 3, wobei die mehreren Leitfähigkeitssensoren (5) in einem Abfluss (6) des Systems (1) positioniert sind, das die Reinigung und Wiederverwendung von Wasser oder die Trennung von Wasser ermöglicht.

5. Verfahren nach einem der Ansprüche 2 bis 3, wobei mindestens ein Leitfähigkeitssensor (5) weiterhin in einem Wassertank (7) des Systems (1) positioniert ist, das die Reinigung und Wiederverwendung von Wasser oder die Trennung von Wasser ermöglicht.

6. Verfahren nach einem der Ansprüche 1 bis 5, wobei jeder der mehreren Leitfähigkeitssensoren (5) auf ein Messen des Wasserpegels oder der Wasserqualität abzielt.

7. Verfahren nach einem der Ansprüche 1 bis 6, wobei die Entladesequenz für jeden spezifischen Sensor (5) der mehreren Leitfähigkeitssensoren (5) Anregen des spezifischen Sensors (5) mit einer Polaritätsseite, Abtasten des spezifischen Sensors (5), Anregen des spezifischen Sensors (5) mit der anderen Polaritätsseite und dann Entladen des spezifischen Sensors (5) umfasst.

8. Verfahren nach Anspruch 7, wobei der erste Schritt des Anregens mit positiver Polarität durchgeführt wird und der zweite Schritt des Anregens mit negativer Polarität durchgeführt wird.

9. Verfahren nach Anspruch 7 oder 8, wobei die Schritte des Anregens in einem Zeitbereich zwischen 20 und 500 µs durchgeführt werden.

10. Verfahren nach einem der Ansprüche 1 bis 9, wobei das System (1) mindestens drei Sensoren (5) umfasst, von denen einer ein Sensor für niedrigen Wasserpegel ist, einer ein Sensor für hohen Wasserpegel ist und ein dritter ein Sensor zur Leitfähigkeitsmessung ist, und wobei das Verfahren erst eine Entladesequenz an dem Sensor für niedrigen Wasserpegel, dann eine Entladesequenz an dem Sensor zur Leitfähigkeitsmessung und schließlich eine Entladesequenz an dem Sensor für hohen Wasserpegel umfasst.

## Revendications

1. Procédé pour mesurer une qualité d'eau dans un système (1), comprenant un système de capteurs (2) avec de multiples capteurs de conductivité (5) situés proches les uns des autres, incluant un capteur de conductivité (5) ordonné de mesurer une qualité d'eau, ledit procédé étant **caractérisé en ce qu'**il comprend une séquence de décharge et d'échantillonnage pour chaque capteur de conductivité (5), dans lequel seulement un capteur de conductivité (5) est mesuré ou échantillonné, un à la fois, pour que, lorsque l'un subit une séquence de décharge et d'échantillonnage, alors l'autre (les autres) est (sont) mis dans une position de repos, correspondant à un état de haute impédance d'électrodes de capteur pour éviter une interférence avec le capteur de conductivité actif (5).

2. Procédé pour faire fonctionner un système (1) permettant l'épuration et le recyclage d'eau ou la séparation d'eau, ledit système (1) comprenant un système de capteurs (2) et un système de commande (3), dans lequel le système de capteurs (2) comprend de multiples capteurs de conductivité (5) situés proches les uns des autres, incluant un capteur de conductivité (5) ordonné de mesurer une qualité d'eau, dans lequel la qualité d'eau dans le système (1) est mesurée avec ledit système de capteurs (2) en utilisant un procédé selon la revendication 1, et dans lequel le système de capteurs (2) fournit une entrée au système de commande (3) en ce qui concerne une décision de sélection de recyclage d'eau dans le système (1) ou de séparation d'eau, à partir du système (1), dans au moins un point de séparation.

3. Procédé selon la revendication 2, dans lequel les multiples capteurs de conductivité (5) sont positionnés à proximité étroite dudit au moins un point de séparation.

4. Procédé selon l'une quelconque des revendications 2 et 3, dans lequel lesdits multiples capteurs de conductivité (5) sont positionnés dans un drain (6) du système (1) permettant l'épuration et le recyclage d'eau ou la séparation d'eau.

5. Procédé selon l'une quelconque des revendications 2 et 3, dans lequel au moins un capteur de conductivité (5) est en outre positionné dans un réservoir d'eau (7) du système (1) permettant l'épuration et le recyclage d'eau ou la séparation d'eau.

6. Procédé selon l'une quelconque des revendications 1 à 5, dans lequel chacun desdits multiples capteurs de conductivité (5) est ordonné de mesurer un niveau d'eau ou une qualité d'eau.

7. Procédé selon l'une quelconque des revendications 1 à 6, dans lequel la séquence de décharge pour chaque capteur spécifique (5) desdits multiples capteurs de conductivité (5) comprend l'excitation du capteur spécifique (5) avec un côté de polarité, l'échantillonnage du capteur spécifique (5), l'excitation du capteur spécifique (5) avec l'autre côté de polarité et puis la décharge du capteur spécifique (5).

8. Procédé selon la revendication 7, dans lequel la première étape d'excitation est réalisée avec une polarité positive et la seconde étape d'excitation est réalisée avec une polarité négative.

9. Procédé selon la revendication 7 ou 8, dans lequel les étapes d'excitation sont réalisées dans une gamme temporelle de 20 à 500 µs.

10. Procédé selon l'une quelconque des revendications 1 à 9, dans lequel le système (1) comprend au moins trois capteurs (5) dont un est un capteur de bas niveau d'eau, un est un capteur de haut niveau d'eau et un troisième est un capteur de mesure de conductivité, et dans lequel le procédé comprend en premier une séquence de décharge sur le capteur de bas niveau d'eau, puis une séquence de décharge sur le capteur de mesure de conductivité et enfin une séquence de décharge sur le capteur de haut niveau d'eau.
